# EUROPEAN PATENT SPECIFICATION

(11) **EP 2 462 888 B1**
(45) Date of publication and mention of the grant of the patent: **18.02.2015**
(21) Application number: 10194607.7
(22) Date of filing: 10.12.2010
(51) Int. Cl.: A61B 17/70

(54) **Receiving part for receiving a rod for coupling the rod to a bone anchoring element and bone anchoring device with such a receiving part**
Aufnahmeteil für die Aufnahme einer Stange zur Kopplung der Stange in einem Knochenverankerungselement und Knochenverankerungsvorrichtung mit einem derartigen Aufnahmeteil
Pièce de réception pour recevoir une tige pour coupler la tige sur un élément d'ancrage d'os et dispositif d'ancrage d'os avec une telle pièce de réception

(43) Date of publication of application: 13.06.2012
(73) Proprietor: Biedermann Technologies GmbH & Co. KG, 78166 Donaueschingen (DE)
(72) Inventor: Biedermann, Lutz, 78048, VS-Villingen (DE); Matthis, Wilfried, 79367, Weisweil (DE)
(74) Representative: Prüfer & Partner GbR European Patent Attorneys

(56) References cited:
- EP-A1- 2 201 903
- EP-A1- 2 204 129
- US-A1- 2005 096 653
- US-A1- 2005 277 927
- US-A1- 2006 200 128

## Description

The invention relates to a receiving part for receiving a rod for coupling the rod to a bone anchoring element and a bone anchoring device with such a receiving part. The head of the bone anchoring element can be locked in the receiving part by compression of a head receiving portion by means of a locking ring, which laterally surrounds the head receiving portion. The head receiving portion has an exterior surface portion and the locking ring has an interior surface portion that engages the exterior surface portion in a contact region to clamp the head. At least one of the exterior and the interior surface portion is cylindrical and the other one is either cylindrical or a ring-shaped bulge. The bone anchoring device can be realized, for example, in the form of a polyaxial bone screw, allowing a pivotal movement of the head and a locking of the head in a desired angular position.

US 5,733,285 describes a polyaxial colletted locking mechanism for use with an orthopaedic apparatus, which includes a screw having a curvate head and a coupling element. The coupling element has a tapered and colletted portion having an interior chamber, in which the curvate head is initially polyaxially disposed. A locking collar is disposed around a tapered and colletted portion, such that translation thereof in the direction of the expanding taper causes the interior volume to contract onto the curvate head and lock it therein. The interior surface of the locking collar also includes a taper.

WO 2007/038350 A2 describes an apparatus for connecting a bone anchor to a support rod that includes a connector body and a cap. The connector body has a channel to receive and locate the support rod relative to the bone anchor. A sleeve fits over a socket portion of the connector body to lock the bone anchor in the socket. The connector body has a lower section with an exterior surface which is at least part-spherical. The sleeve has a conical inner surface, which is slideable over the outer surface of the lower section of the socket to compress the socket to lock the bone anchor relative to the connector body.

Document US 2005/0096653 A1 describes an orthopaedic device. The device comprises clamps respectively having a slot for gripping a connector rod. The clamps further comprise receptacles for accommodating the head of a bone screw, the receptacles have an open end and the clamps are provided with circumferential slots to increase the radial flexibility of the clamps. The exterior walls of the clamps are shaped to provide a change in external diameter along the length. Each of the clamps include a compression ring embracing the clamps and tightened to hold a rod connecting several screws through slots in the clamps by linear movement of the compression rings.

Document US 2006/0300128 A1 describes a spinal rod bone anchor for engaging a rod with a lock assembly. The lock assembly may include a bottom ring surrounding a lower portion of the lock assembly. When the bottom ring is translated longitudinally toward the bone, the anchor is clamped by means of fingers, on which a radial force directed inwardly is applied by the translation.

Document EP 2 204 129 A1 describes a receiving part for receiving a rod for coupling the rod to a bone anchoring element. The receiving part includes a locking ring having a tapered inner surface. At the lower portion of a receiving part body, the receiving part is provided with a tapered outer surface. When the locking ring is moved towards the bone, a head of the bone anchoring element is clamped.

Document EP 2 201 903 A1 also describes a receiving part for receiving a rod for coupling the rod to a bone anchoring element. At the lower portion, the receiving part is provided with two curved exterior surface portions having different outer diameters. The locking ring is provided with two curved interior surface portions cooperating for clamping the bone anchoring element when the locking ring is moved towards the bone.

Document US 2005/0277927 A1 describes a fastening system for a spinal stabilization system. The fastening system may have a locking sleeve which, when moved toward the bone, pushes the rod which is accommodated in a rod receiving pocket against a retainer of a head of a bone anchoring element. The retainer is located in an anchor-receiving pocket having a tapered internal surface. By pushing the retainer towards the bone, the head of the bone anchoring element is clamped by the tapered internal surface. In its end position the locking sleeve engages with locking structures of legs forming the rod receiving pocket.

US 2009/0149887 describes an apparatus for connecting a bone anchor to a support rod that includes a connector body and a cap. A sleeve fits over a socket portion of the connector body. The sleeve is movable between a temporary position in which the sleeve permits insertion of the bone anchor, a provisional locking position in which the sleeve permits polyaxial angulation of the bone anchor with respect to the connector body but prevents removal of the bone anchor from the connector body and a locking position in which the sleeve prevents both, polyaxial angulation and removal of the bone anchor with respect to the connector body.

It is an object of the invention to provide a receiving part for receiving a rod for coupling the rod to a bone anchoring element and a bone anchoring device with such a receiving part, which is improved with respect to safety in use.

The object is solved by a bone anchoring device according to claim 1. Further developments are given in the dependent claims.

The bone anchoring device has an improved safety since it exhibits a greater locking force compared to a bone anchoring device which clamps the head using at least one tapered surface portion at the head receiving portion or the locking ring. The greater locking force is achieved in that the cooperating surfaces of the receiving part and the locking ring are configured to create a clamping force during vertical movement on the locking ring that is perpendicular to the central axis of the receiving part.

The enhanced locking force is achieved with a compact and low profile design of the bone anchoring device, in particular with respect to a maximum outer diameter of the bone anchoring device. Furthermore, the bone anchoring device consists of simple mechanical elements that can be easily manufactured. The bone anchoring device may also have a pre-locking function, which is characterized in that the locking ring holds the head in the head receiving portion such that it can pivot but cannot be removed from the head receiving portion.

In addition, the head may be held in the head receiving portion by applying a small pre-load to frictionally clamp the head, so that it can only pivot by applying a force greater than the friction force.

Furthermore, with the bone anchoring device a modular system can be provided with various bone anchors, that can be mounted to the receiving part easily prior to surgery.

Further features and advantages of the invention will become apparent from the description of embodiments using the accompanying drawings.

In the drawings:
- Fig. 1:: shows a perspective exploded view of a first embodiment of the bone anchoring device.
- Fig. 2:: shows a perspective view of the bone anchoring device of Fig. 1 in an assembled state.
- Fig. 3:: shows a perspective view of the receiving part body of the bone anchoring device according to the first embodiment.
- Fig. 4:: shows a side view of the receiving part body of Fig. 3.
- Fig. 5:: shows a bottom view of the receiving part body of Fig. 3.
- Fig. 6:: shows a perspective view of the locking ring of the bone anchoring device of the first embodiment.
- Fig. 7:: shows a side view of the locking ring of Fig. 6.
- Fig. 8:: shows a sectional cross-sectional view of the locking ring of Figs. 6 and 7 along line A-A in Fig. 7.
- Figs. 9a):: shows cross-sectional view of the bone anchoring device of the first embodiment in a first step of use in which the head can be introduced into the receiving part.
- Fig. 9b):: shows a cross-section view of the bone anchoring device of the first embodiment in a second step of use in which the head is introduced into receiving part and the locking ring is in a pre-locking position.
- Fig. 9c):: shows a cross-sectional view of the bone anchoring device with a rod inserted and the angular position of the screw is locked by the locking ring in the locking position.
- Fig. 9d):: shows an enlarged portion of Fig. 9c).
- Fig. 10:: shows an enlarged section of a cross-sectional view of the bone anchoring device according to a second embodiment.
- Fig. 11:: shows an enlarged section of a cross-sectional view of the bone anchoring device according to a third embodiment.

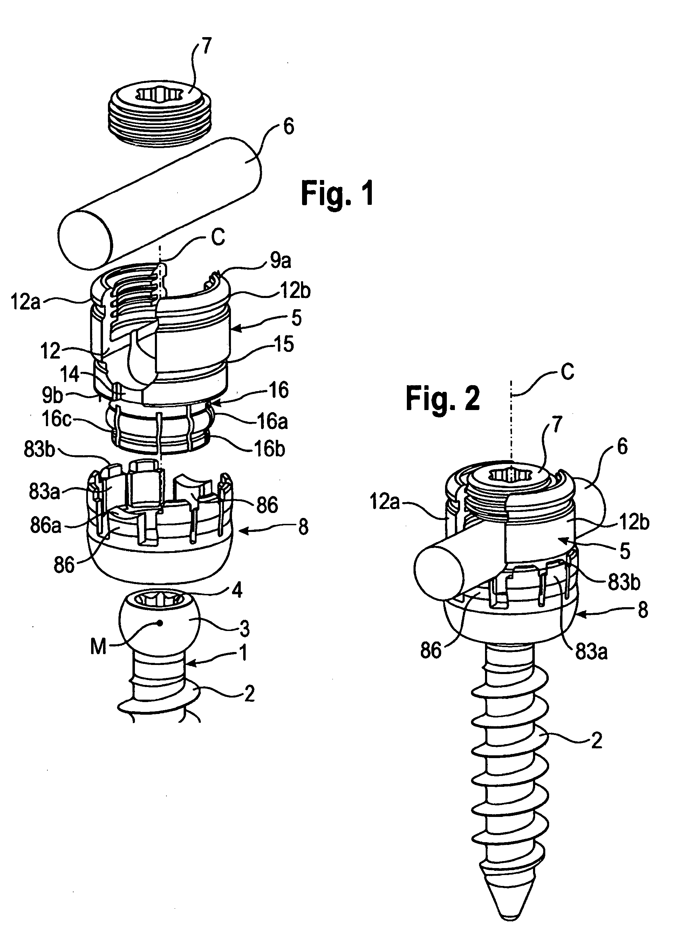

As shown in Figs. 1 and 2, the bone anchoring device according to a first embodiment includes a bone anchoring element 1 in the form of a bone screw having a threaded shaft 2 and a spherical segment-shaped head 3 with a center M of the sphere. The head 3 has a recess 4 for engagement with a driver. The bone anchoring device further includes a receiving part body 5 for receiving a rod 6 to connect the rod to the bone anchoring element 1. Further, a closure element 7 in the form of an inner screw or set screw is provided for securing the rod 6 in the receiving part. In addition, the bone anchoring device includes a locking ring 8 for locking the head 3 in the receiving part body 5. The receiving part body 5 and the locking rings form a receiving part.

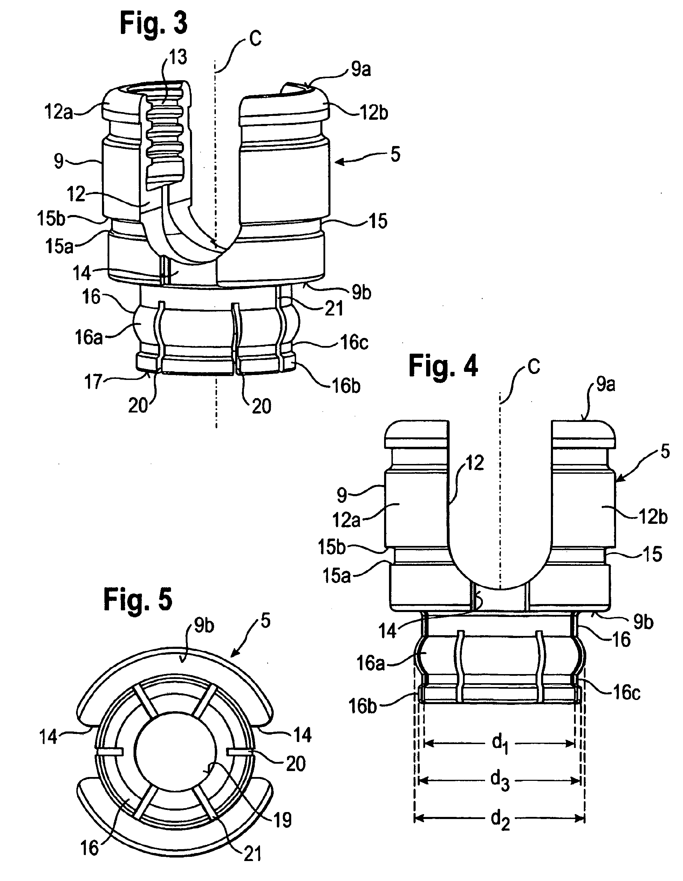

As further shown in Figs. 3 to 6 and 9a) to 9c), the receiving part body 5 comprises a rod receiving portion 9, which has a first end 9a and an opposite second end 9b. The rod receiving portion 9 is substantially cylindrical and comprises a coaxial first bore 10 extending from the first end 9a to the second end 9b and a central axis C. The diameter of the first bore 10 is smaller than the greatest diameter of the head 3 of the bone anchoring element 1. The rod receiving portion 9 further comprises a coaxial second bore 11 extending from the first end 9a to a distance from the second end 9b. The diameter of the second bore 11 is larger than that of the first bore 10 and larger than the diameter of the rod 6. A U-shaped recess 12 is provided in the rod receiving portion 9 that extends from the first end 9a in the direction of the second end 9b. The U-shaped recess 12 forms a channel for the rod which has a size such that the rod 6 can be placed and guided therein. By means of the U-shaped recess 12, two free legs 12a, 12b are formed on which an internal thread 13 is provided. The internal thread can be a metric thread, a flat thread, a negative angle thread, a saw-tooth thread or any other thread form. Preferably, a thread form such as a flat thread or a negative angle thread is used, which prevents splaying of the legs 12a, 12b when the inner screw 7 is screwed-in. The depth of the U-shaped recess 12 is such that the rod 6 and the inner screw 7 can be inserted between the legs 12a, 12b.

At either end of the U-shaped recess 12 seen in a direction along the channel axis, cutouts 14 extend from the second end 9b into the bottom of the U-shaped recess 12.,

In the outer surface of the rod receiving portion 9 in the region of the legs 12a, 12b a groove 15 is provided, which extends in a circumferential direction and serves for engagement with a portion of the locking ring 8. The groove 15 is asymmetric in such a way, that it allows a disengagement of the locking ring and the groove, when the locking 8 is shifted downwards. The asymmetric shape is realized by a downwardly inclined lower wall 15a and a substantially perpendicular upper wall 15b of the groove. Between the groove 15 and the second end the diameter of the rod receiving portion 9 may be slightly reduced.

Adjacent to the second end 9b, the receiving part 5 includes a head receiving portion 16 providing an accommodation space for the head 3 of the bone anchoring element 1. The head receiving portion 16 has a free end 17 opposite to the second end 9b of the rod receiving portion 9. A maximum outer diameter of the head receiving portion 16 is smaller than the outer diameter of the rod receiving portion 9, so that the head receiving portion 16 is recessed with respect to the rod receiving portion 9. The head receiving portion 16 has an internal hollow section 18, which is spherically-shaped with a radius matching the radius of the head 3 of the bone anchoring element. The internal hollow section 18 has an opening 19 at the free end 17. The dimension of the internal hollow section 18 is such that the hollow section 18 encompasses the head 3 of the bone anchoring element including a region of the largest diameter of the head 3. When the head is introduced into the internal hollow section 18 its center M lies substantially on the central axis.

Slits 20 are provided in the head receiving portion 16, which are open to the free end 17 and extend to a distance from the second end 9b of the head receiving portion. The slits 20 have at least such a length that they include the region of the largest inner diameter of the internal hollow section 18. Other slits 21 extend from the free end 17 to the second end 9b of the rod receiving portion. The slits in the head receiving portion 16 render the head receiving portion 16 flexible, such that the head 3 of the anchoring element can be inserted by expanding the head receiving portion and can be clamped by compressing the head receiving portion.

As best seen in Fig. 4, the outer surface of the head receiving portion is generally cylindrical with an outer diameter d₁. At a distance from the second end 9b of the rod receiving portion an exterior surface portion in the form a ring-shaped bulge 16a with a largest outer diameter d₂ greater than d₁ is provided. The ring-shaped bulge 16a includes a region of the largest inner diameter of the internal hollow section 18. The cross-section of the ring-shaped bulge 16a may be a section of a circle, however, it can also be otherwise shaped, for example it can be a section of an ellipsoid or can be asymmetric in an axial direction. Adjacent to the free end 17, a cylindrical portion 16b is formed that has an outer diameter d₃ greater than d₁ and smaller than or equal to d₂. Hence, between the ring-shaped bulge 16a and the cylindrical portion 16b, a groove 16c is formed. The upper edge of the cylindrical section 16b may be chamfered to facilitate engagement with the locking ring 8 described below.

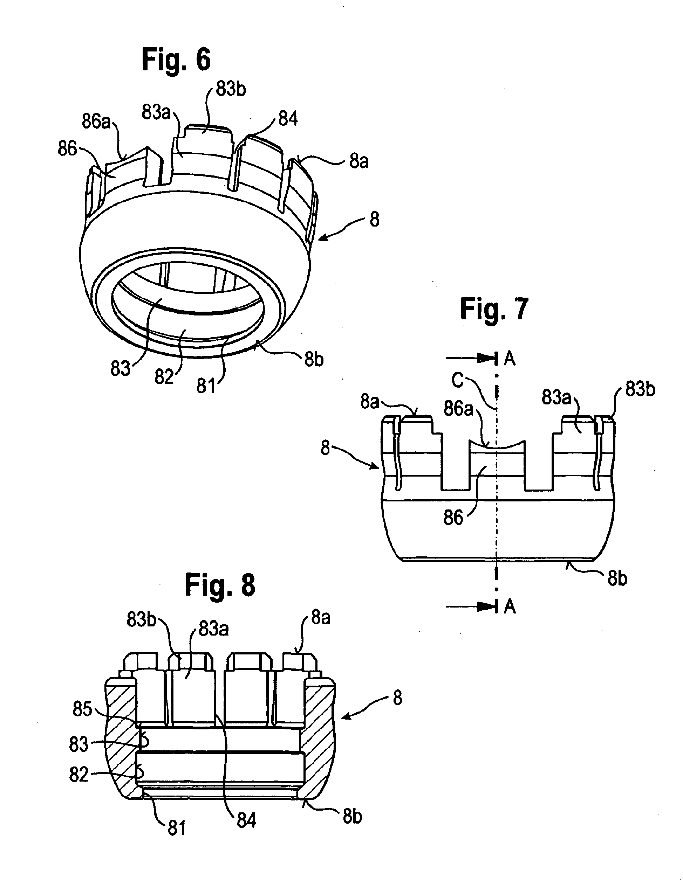

The locking ring will now be described with reference to Figs. 6 to 9a) to 9c). The locking ring 8 is substantially cylindrical and has an upper end 8a and a lower end 8b. In the mounted state, the upper end 8a is oriented in the direction of the first end 9a of the rod receiving portion 9 and the lower end 8b is oriented towards the free end 17 of the head receiving portion 16.

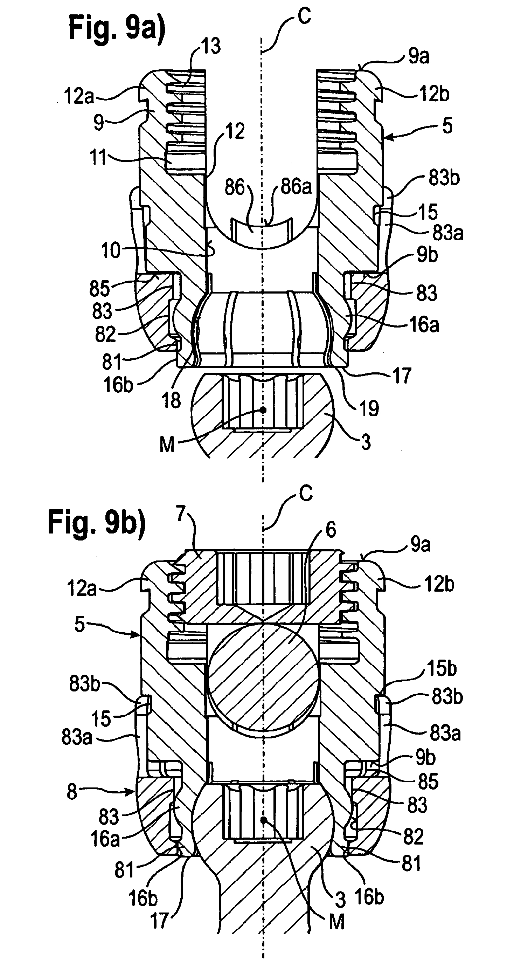

Adjacent the lower end 8b, the locking ring 8 has a first hollow cylindrical portion 81 with an inner diameter that is slightly smaller than the outer diameter of the cylindrical portion 16b of the head receiving portion 16 in such a way that the cylindrical portion 81 and the cylindrical portion 16b of the head receiving portion can engage in an interference-fit connection. The height of the first hollow cylindrical portion 81 is such that the portion 81 can engage the groove 16c of the head receiving portion. Following the first hollow cylindrical portion 81, there is a second hollow cylindrical portion 82 with an inner diameter that is greater than the outer diameter d₂ of the ring-shaped bulge 16a of the head receiving portion 16, to such an extent that in a first position of the locking ring 8, which is shown in Fig. 9a), the ring-shaped bulge 16a can extend into the second hollow cylindrical portion 82 of the locking ring and has space for expansion in a radial direction. The height of the second hollow cylindrical portion 82 is about the height of the ring-shaped bulge 16a in an axial direction.

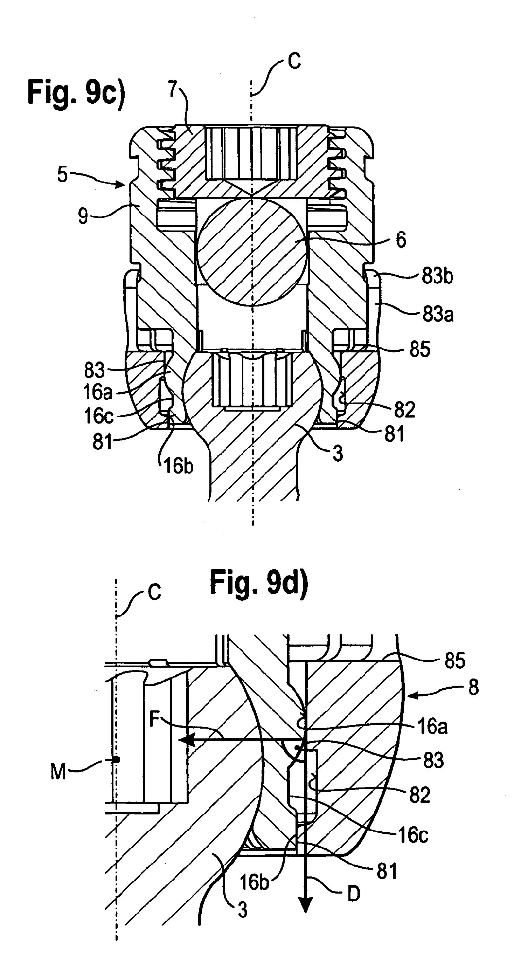

Following the second hollow cylindrical portion 82 a third hollow cylindrical portion 83 is provided. The inner diameter of the third hollow cylindrical portion 83 is slightly smaller than the greatest outer diameter d₂ of the ring-shaped bulge of 16a of the head receiving portion, such that when the third cylindrical portion 83 cooperates with the ring-shaped bulge 16a an interference-fit connection is achieved. The height of the third hollow cylindrical portion 83 is such that when the locking ring is in its lowermost position shown in Fig. 9c), the third hollow cylindrical portion 83 is in contact with at least the area of the greatest outer diameter of the ring-shaped bulge 16a.

The locking ring 8 further comprises upwardly extending flexible wall portions 83a which are separated from each other by slits 84. The upwardly extending wall portions 83a are arranged at the outer circumference of an inner circumferential shoulder 85 of the locking ring and render the locking ring flexible in its upper region. The number and size of the slits and the thickness of the wall portions 83a are configured such that a desired flexibility is obtained. At the free end the wall portions 83a comprise engagement sections 83b, which are shaped so as to engage the groove 15 provided on the outer surface of the rod receiving portion 9.

Two projections 86, which are located diametrically opposite to each other, extend from the shoulder 85. The projections 86 have such a height that they project into the bottom of the U-shaped recess 12 through the cutouts 14, when the locking ring 8 is in a position in which the head is not yet locked as shown in Figs. 9a) and 9b). The free end surface 86a of the projections 86 can be curved, for example with a curvature corresponding to the curvature of the rod 6. The locking ring 8 is arranged around the head receiving portion 16 with the projections 86 located at the positions of the U-shaped recess 12. By means of this, the projections 86 prevent the locking ring 8 from rotating, when the rod is not inserted.

The flexibility of the head receiving portion 16 and the size of the head receiving portion at the open end 17 allows to mount the locking ring by assembling it from the free end 17 onto the head receiving portion 16. The outer diameter of the locking ring 8 may be designed such that the locking ring 8 does not project substantially beyond the rod receiving portion 9 of the receiving part to ensure a compact shape.

The inner screw 7 has a thread corresponding to the internal thread 13 provided on the legs 12a, 12b. If a thread form that prevents the legs from splaying is used, a single fixation element such as the inner screw 7 is sufficient. This reduces the size of the bone anchoring device in a radial direction.

The receiving pant 5, the locking ring 8, the inner screw 7 and the bone anchoring element 1 are made of a bio-compatible material, for example of titanium or of stainless steel or of bio-compatible allows such as Nitinol or of a bio-compatible plastic material, such as polyether-etherketone (PEEK). The part can be made all of the same or of different materials.

The function of the locking ring 8 will now be explained with reference to Figs. 9a) to 9c). As shown in Fig. 9a), a first position of the locking ring 8, which is the insertion position and in which the locking ring 8 is latched with respect to the receiving part 5, is defined in such a way that the first hollow cylindrical portion 81 engages the groove 16c at the outer surface of the head receiving portion 16. Since the inner diameter of the first hollow cylindrical portion 81 and the inner diameter of the second hollow cylindrical portion 82 are larger than the outer diameter of the head receiving portion 16 at the position of the groove 16c and of the ring-shaped bulge 16a, respectively, an expansion of the head receiving portion 16 is possible, when the head is introduced. In a first position, the locking ring 8 is additionally held by a clamping force between the rod receiving portion 9 and the flexible wall portions 83a which are slightly bent outwards as can be seen in Fig. 9a).

When the locking ring 8 is in the first position, the head receiving portion 16 is not compressed. In this position, the introduction of the screw head 3 is possible as can be seen in Fig. 9a). In the first position, the locking ring 8 is prevented from moving further upwards towards the first end 9a) of the rod receiving portion, since it abuts with the shoulder 85 against the second end 9b of the rod receiving portion. The inclined lower wall 15a of the groove 15 prevents and inadvertent downward movement of the locking ring 8 but allows downward movement of the locking ring upon exertion of a force.

After introduction of the head 3, the locking ring 8 is moved downwards in an axial direction into a second position, in which it is latched with respect to the receiving part and which is a pre-locking position, as shown in Fig. 9b). In the second position, the locking ring 8 has been shifted towards the free end 17 of the head receiving portion 16, until the engagement portions 83b of the flexible wall portions 83a resiliently snap into the groove 15 provided at the rod receiving portion 9. The free upper edge of the engagement portions 83b abut against the upper wall 15b of the groove 15, thereby preventing upward movement of the locking ring out of the pre-locking position. On the other hand, the inclined lower wall 15a of the groove 15 prevents inadvertent downward movement of the locking ring towards the free end 17 that allows such downward movement upon exertion of a force.

In the second position as shown in Fig. 5b), the first hollow cylindrical portion 81 slightly compresses the cylindrical portion 16b of the head receiving portion in an interference-fit manner, so that the head 3 is prevented from removal out of the hollow internal section 18. The second hollow cylindrical portion 82 begins to engage the ring-shaped bulge 16a, so as to slightly compress the head receiving portion 16, thereby further contributing to prevention of removal of the head 3. In addition, if desired, the dimensions of the engaging portions can be defined such that in the second position, as shown in Fig. 9b), the head 3 is held in the hollow internal section 18 by friction in an adjustable angular position, so that it can still be pivoted by applying a force greater than the friction force.

A third position, which is the locking position, is shown in Fig. 9c) where the locking ring has been moved further axially downwards. The third position is defined as the position, in which the screw head 3 is finally locked within the head receiving portion 16. The inner surfaces of the first hollow cylindrical portion 81 and the third hollow cylindrical portion 83 engage the cylindrical portion 16b and the ring-shaped bulge 16a in an interference-fit manner, thereby firmly compressing the head receiving portion 16 around the head 3. The head 3 is locked by exerting the pressure at two defined circumferential areas, one at the ring-shaped bulge, which is slightly above the area with the greatest diameter of the head 3 and the other at the cylindrical portion 16b, which is below the area of the greatest diameter of the head 3. The clamping force as shown in Fig. 9d by the arrow F that acts onto the head receiving portion when the locking ring is moved in the direction indicated by arrow D is perpendicular to the central axis C of the receiving part. Thereby, the force acting in the locking position onto the head is enhanced compared to a device that uses tapered surface portions. The movement of the cooperating surfaces is a vertical movement coaxial to the central axis. Therefore, the force flow is predictable. The shapes of the cooperating surfaces are easy to manufacture.

The bone anchoring device may be pre-assembled as follows. First, the locking ring 8 is mounted onto the receiving part from the free end 17. This can be done, for example, by the manufacturer. Preferably, the locking ring 8 is in the insertion position. Thereafter, the head 3 of the anchoring element 1 is introduced from the free end into the hollow internal portion 18 of the head receiving portion 16. Thereafter, the locking ring is moved downwards into the pre-locking position.

During surgery, the pre-assembled bone anchoring device is screwed into the bone. The recess 4 of the head 3 can be accessed with a driver through the first bore 10. If the head 3 is held in an adjustable angular position by friction in the pre-locking position, an additional force is exerted onto the receiving part either manually or by application of an instrument to align the receiving part with respect to the rod. Once the correct position of the rod with respect to other bone anchoring devices is achieved, the inner screw 7 is tightened. When the rod 6 abuts onto the projections 86 of the locking ring, the locking ring 8 is shifted downwards into the locking position. Final tightening of the inner screw locks the rod and the head simultaneously.

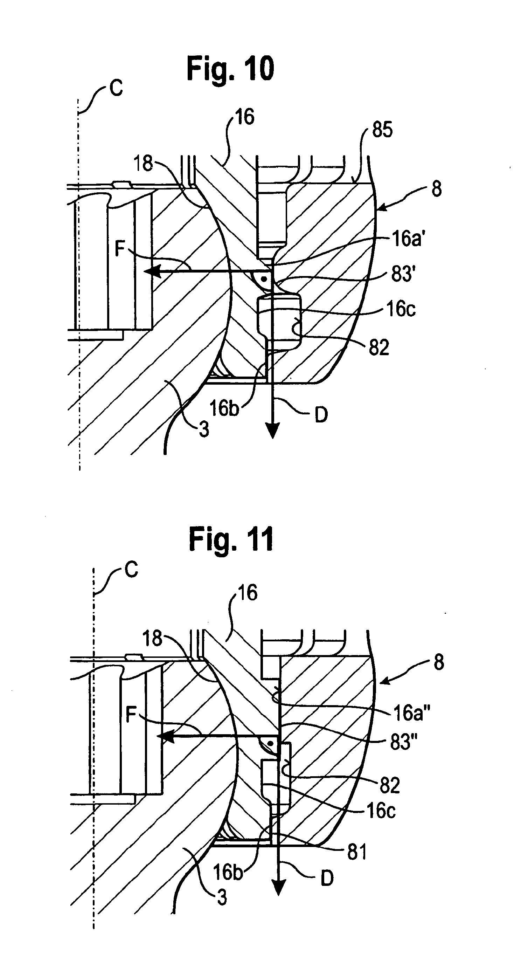

A second embodiment of the bone anchoring device is shown in Fig. 10.

The bone anchoring device according to the second embodiment differs from the bone anchoring device according to the first embodiment only in the design of the exterior surface of the head receiving portion 16 and the interior surface of the locking ring 8. All other parts are the same and are designated with the same reference numerals and the description thereof is not repeated. The head receiving portion 16 has instead of the ring-shaped bulge 16a, a cylindrical surface portion 16a' with a greatest outer diameter d₂ which is greater than the outer diameter d₁ of the cylindrical head receiving portion 16. The locking ring 8 comprises instead of the third hollow cylindrical portion 83 an inwardly projecting ring-shaped bulge 83' with an inner diameter that is slightly smaller than the outer diameter of the cylindrical portion 16a' of the head receiving portion 16 to enable an interference-fit between the two portions. Hence, the cooperating exterior and interior surface portions are reversed with respect to those of the first embodiment. Also in this embodiment, the locking of the head is achieved by an interference-fit between the engaging exterior surface portion of the head receiving portion and the interior surface portions of the locking ring at two contact areas that are approximately around the region with the largest diameter of the head or above and below the region of the largest diameter of the head. Also in this embodiment, the clamping force F is directed perpendicular to the central axis of the receiver resulting in a high locking force in the locking position.

A third embodiment is shown in Fig. 11. Parts which are identical to that of the first and second embodiments are designated with the same reference numerals and the description thereof is not repeated. The bone anchoring device of the third embodiment differs from the bone anchoring device of the first and second embodiment in the shape of the exterior surface of the head receiving portion 16 and the interior surface of the locking ring 8. Instead of an engagement between a cylindrical surface and a ring-shaped bulge, an engagement between a first cylindrical surface and a second cylindrical surface is provided. The head receiving portion 16 has a cylindrical surface portion 16a" with an outer diameter d₂ that is larger than the outer diameter of the cylindrical portion in the groove 16c". The cylindrical portion 16a" extends in an axial direction over an area that includes the region of the largest outer diameter of the head 3. The locking ring 8 has a third cylindrical portion 83" with an inner diameter that is slightly smaller than the outer diameter of the cylindrical portion 16a" to allow an interference-fit between the portion 16a" and the portion 83". The axial length of the third hollow cylindrical portion 83" is such that it can come into engagement with the cylindrical portion 16a" over a substantial area including the region with the largest outer diameter of the head 3. The locking of the head in the third embodiment is achieved by exerting a force F onto the head receiving portion 16 via an interference-fit of cylindrical surfaces. Also in this embodiment, the clamping force is perpendicular to the central axis C. Hence, the locking force that can be achieved is greater than the locking force that can be achieved with engaging surfaces including a tapered portion.

Modifications of the embodiments are possible. For example, the lower engaging surfaces at the free end 17 of the head receiving portion with the locking ring can be omitted. That means for locking the head it is sufficient to provide engaging surfaces in only one circumferential area. In this case, the engaging surfaces should cover the region of the largest diameter of the head. The pre-locking function can be realized otherwise by engagement of portions of the locking ring with other portions of the receiving part. The ring-shaped bulge portion may also have interruptions in a circumferential direction.

The shape of the receiving part body is not limited to the embodiment shown. For example, in the rod receiving portion, there can also be slits provided.

The head of the bone anchoring element can have any other shape, such as, for example, a cylindrical shape, whereby a monoaxial bone screw is provided allowing rotation of the screw element with respect to the receiving part around a single axis.

The projections of the locking ring that engage the rod, can have another shape, for example, the surface of the free end of the projection can be flat or can be otherwise shaped. The projections can also be omitted.

The head receiving portion can have an inclined open end or can be otherwise asymmetric to allow a greater angulation of the head in one direction.

The cylindrical surface portion 16b and the first hollow cylindrical portion 81 can also be replaced by ring-shaped bulges.

For the bone anchoring element, all kinds of anchoring elements can be used and combined with the receiving part. These anchoring elements are e.g, screws of different length, with different diameters, cannulated screws, screws with different thread forms, nails, etc. The head of the screw and the shaft can also be separate parts, which are connectable to each other.

The channel for the rod can be closed on the first end of the rod receiving portion. For the closure element other types such as outer nuts or other closure elements can be used.

## Claims

1. A receiving part for receiving a rod for coupling the rod to a bone anchoring element, the receiving part including
a receiving part body (5) with
a rod receiving portion (9) with a channel (12) for receiving the rod, and
a head receiving portion (16) with an outer surface that is generally cylindrical and has an outer diameter (d₁) for accommodating a head (3) of the bone anchoring element, the head receiving portion having an open end (17) and being flexible, so as to allow introduction and clamping of the head, the head receiving portion (16) having a first exterior surface portion (16a, 16a', 16a") with a largest outer diameter (d₂) greater than the outer diameter (d₁) of the head receiving portion (16), a cylindrical portion (16b) adjacent to the open end (17) and having an outer diameter (d₃) greater than the outer diameter (d₁) of the head receiving portion (16) and smaller than the outer diameter (d₂) of the first exterior surface portion (16a, 16a', 16a"), and a central axis (C) extending through the open end (17); and
a locking ring (8) embracing the head receiving portion (16), the locking ring having a first interior surface portion (83, 83', 83") that engages the first exterior surface portion (16a, 16a', 16a") in a first contact region to clamp the head (3) and a first hollow cylindrical portion (81), adjacent a lower end (8b) of the locking ring (8), with an inner diameter that is slightly smaller than the outer diameter (d₃) of the cylindrical portion (16b) for engaging in an interference-fit connection;
wherein at least one of the exterior and the interior surface portions is a cylindrical surface portion (83, 16a") and the other one is either a cylindrical surface portion (83", 16a") or a ring-shaped bulge portion (16a, 83') and wherein the locking ring (8) is movable towards the open end (17) upon exerting a pressure onto it via the rod (6) to clamp the head.

2. The receiving part of claim 1, wherein an inner diameter of the first interior surface portion (83, 83', 83") at the first contact region is smaller than the outer diameter of the first exterior surface portion (16a, 16a', 16a") at the first contact region to achieve an interference-fit connection.

3. The receiving part of claim 1 or 2, wherein the head receiving portion (16) has the cylindrical portion (16b) as a second exterior surface portion that is separated from the first exterior surface portion (16a, 16a', 16a") in an axial direction and such engages the first hollow cylindrical portion (81) as a second interior surface portion of the locking ring that is separated from the first interior surface portion (83, 83', 83") in an axial direction to form a second contact region.

4. The receiving part of claim 3, wherein the second exterior surface portion is a cylindrical surface portion (16b).

5. The receiving part of claim 3 or 4, wherein the second interior surface portion is a cylindrical surface portion (81).

6. The receiving part of one of claims 3 to 5, wherein the second contact region is closer to the central axis (C) in a radial direction than the first contact region.

7. The receiving part of one of claims 3 to 5, wherein the second contact region has the same distance from the central axis (C) as the first contact region.

8. The receiving part of one of claims 3 to 7, wherein the second contact region is adjacent to the open end (17).

9. The receiving part of one of claims 1 to 8, wherein the first exterior surface portion (16a") and the second exterior surface portion (83") are both cylindrical surface portions.

10. The receiving part of one of claims 1 to 8, wherein the first exterior surface portion (16a) is a ring-shaped bulge portion and the first interior surface portion (83) is a cylindrical surface portion.

11. The receiving part of one of claims 1 to 8, wherein the first exterior surface portion (16a') is a cylindrical surface portion and the first interior surface portion (83') is a ring-shaped bulge portion.

12. The receiving part of one of claims 3 to 11, wherein a groove (16c) is provided between the first exterior surface portion (16a, 16a', 16a") and a second exterior surface portion (83,83', 83") into which the locking ring can engage when it is an insertion position, in which the head is insertable into and removable from the head receiving portion (16).

13. A receiving part of one of claims 1 to 12, wherein the locking ring (8) can assume a pre-locking position, in which the head is pivotable in the head receiving portion but not removable from the head receiving portion.

14. The receiving part of one of claims 1 to 13, wherein the locking ring (8) can assume a locking position, in which the first exterior surface portion (16a, 16a', 16a") contacts the first interior surface portion (83, 83', 83").

15. The receiving part of one of claims 1 to 14, wherein the rod receiving portion (9) has a first end (9a) and a second end (9b) and a recess (12) extending from the first end in the direction of the second end, which forms the channel for the rod.

16. The receiving part of one of claims 1 to 15, wherein the rod receiving portion (9) has a first end (9a) and a second end (9b) and a head receiving portion (16) is arranged at the side of the second end (9b) and wherein the outer diameter of the head receiving portion (16) at the side of the second end (9b) is smaller than the diameter of the rod receiving portion (9) at the second end (9b).

17. A bone anchoring device comprising a receiving part according to one of claims 1 to 16 and a bone anchoring element (1) having a shaft (2) for anchoring in a bone and a head (3) and wherein the head (3) has preferably an exterior outer surface portion, which is spherically-shaped.

18. The bone anchoring device according to claim 17, wherein a closure clement (7), preferably an inner screw, is provided for securing the rod (6) in a recess (12).

## Patentansprüche

1. Aufnahmeteil zum Aufnehmen eines Stabs zum Koppeln des Stabs an ein Knochenverankerungselement, wobei das Aufnahmeteil
einen Aufnahmeteilkörper (5) mit
einem Stabaufnahmeabschnitt (9) mit einem Kanal (12) zum Aufnehmen des Stabs, und
einem Kopfaufnahmeabschnitt (16), zum Aufnehmen eines Kopfs (3) des Knochenverankerungselements, mit einer äußeren Fläche, die weitgehend zylindrisch ist und einen äußeren Durchmesser (d₁) hat, wobei der Kopfaufnahmeabschnitt ein offenes Ende (17) hat und flexibel ist, um so ein Einführen und Klemmen des Kopfes zu ermöglichen, der Kopfaufnahmeabschnitt (16) einen ersten äußeren Flächenabschnitt (16a, 16a', 16a") mit einem größten äußeren Durchmesser (d₂), der größer als der äußere Durchmesser (d₁) des Kopfaufnahmeabschnitts (16) ist, angrenzend an das offene Ende (17) einen zylindrischen Abschnitt (16b), der einen äußeren Durchmesser (d₃) hat, der größer als der äußere Durchmesser (d₁) des Kopfaufnahmeabschnitts (16) und kleiner als der äußere Durchmesser (d₂) des ersten äußeren Flächenabschnitts (16a, 16a', 16a") ist, und eine Mittelachse (C) hat, die sich durch das offenen Ende (17) erstreckt;
und einen Feststellring (8) enthält, der den Kopfaufnahmeabschnitt (16) umschließt, wobei der Feststellring einen ersten inneren Flächenabschnitt (83, 83', 83"), der an den ersten äußeren Flächenabschnitt (16a, 16a', 16a") in einem ersten Kontaktbereich angreift, um den Kopf (3) zu klemmen, und, angrenzend an ein unteres Ende (8b) des Feststellrings (8), einen ersten hohlen zylindrischen Abschnitt (81) mit einem inneren Durchmesser hat, der zum Angreifen in einer Presssitzverbindung geringfügig kleiner als der äußere Durchmesser (d₃) des zylindrischen Abschnitts (16b) ist;
wobei zumindest einer von dem äußeren und dem inneren Flächenabschnitt ein zylindrischer Flächenabschnitt (83, 16a") und der andere entweder ein zylinderförmiger Flächenabschnitt (83", 16a") oder ein ringförmiger Wulstabschnitt (16a, 83') ist, und wobei der Feststellring (8) über ein Aufbringen eines Drucks über den Stab (6) auf ihn zu dem offenen Ende (17) hin bewegbar ist, um den Kopf zu klemmen.

2. Aufnahmeteil von Anspruch 1, wobei ein innerer Durchmesser des ersten inneren Flächenabschnitts (83, 83', 83") an dem ersten Kontaktbereich kleiner als der äußere Durchmesser des ersten äußeren Flächenabschnitts (16a, 16a', 16a") an dem ersten Kontaktbereich ist, um eine Presssitzverbindung zu erzielen.

3. Aufnahmeteil von Anspruch 1 oder 2, wobei der Kopfaufnahmeabschnitt (16) den zylindrischen Abschnitt (16b) als einen zweiten äußeren Flächenabschnitt hat, der in einer axialen Richtung von dem ersten äußeren Flächenabschnitt (16a, 16a', 16a") getrennt ist, und so an den ersten hohlen zylindrischen Abschnitt (81) als einen zweiten inneren Flächenabschnitt des Feststellrings, der von dem ersten inneren Flächenabschnitt (83, 83', 83") in einer axialen Richtung getrennt ist, angreift, um einen zweiten Kontaktbereich zu bilden.

4. Aufnahmeteil von Anspruch 3, wobei der zweite äußere Flächenabschnitt ein zylindrischer Flächenabschnitt (16b) ist.

5. Aufnahmeteil von Anspruch 3 oder 4, wobei der zweite innere Flächenabschnitt ein zylindrischer Flächenabschnitt (81) ist.

6. Aufnahmeteil von einem der Ansprüche 3 bis 5, wobei der zweite Kontaktbereich in einer radialen Richtung näher an der Mittelachse (C) als der erste Kontaktbereich ist.

7. Aufnahmeteil von einem der Ansprüche 3 bis 5, wobei der zweite Kontaktbereich den gleichen Abstand von der Mittelachse (C) wie der erste Kontaktbereich hat.

8. Aufnahmeteil von einem der Ansprüche 3 bis 7, wobei der zweite Kontaktbereich an das offene Ende (17) angrenzend ist.

9. Aufnahmeteil von einem der Ansprüche 1 bis 8, wobei sowohl der erste äußere Flächenabschnitt (16a") als auch der zweite äußere Flächenabschnitt (83") zylindrische Flächenabschnitte sind.

10. Aufnahmeteil von einem der Ansprüche 1 bis 8, wobei der erste äußere Flächenabschnitt (16a) ein ringförmiger Wulstabschnitt ist, und der erste innere Flächenabschnitt (83) ein zylindrischer Flächenabschnitt ist.

11. Aufnahmeteil von einem der Ansprüche 1 bis 8, wobei der erste äußere Flächenabschnitt (16a') ein zylindrischer Flächenabschnitt ist und der erste innerer Flächenabschnitt (83') ein ringförmiger Wulstabschnitt ist.

12. Aufnahmeteil von einem der Ansprüche 3 bis 11, wobei eine Nut (16c) zwischen dem ersten äußeren Flächenabschnitt (16a, 16a', 16a") und einem zweiten äußeren Flächenabschnitt (83, 83', 83") vorgesehen ist, in den der Feststellring eingreifen kann wenn er in einer Einführposition ist, in der der Kopf in den Kopfaufnahmeabschnitt (16) einführbar und davon entfernbar ist.

13. Aufnahmeteil von einem der Ansprüche 1 bis 12, wobei der Feststellring (8) eine Vor-Feststellposition annehmen kann, in der der Kopf in dem Kopfaufnahmeabschnitt schwenkbar aber nicht aus dem Kopfaufnahmeabschnitt entfernbar ist.

14. Aufnahmeteil von einem der Ansprüche 1 bis 13, wobei der Feststellring (8) eine Feststellposition annehmen kann, in der der erste äußere Flächenabschnitt (16a, 16a', 16a") den ersten inneren Flächenabschnitt (83, 83', 83") berührt.

15. Aufnahmeteil von einem der Ansprüche 1 bis 14, wobei der Stabaufnahmeabschnitt (9) ein erstes Ende (9a) und ein zweites Ende (9b) und eine sich von dem ersten Ende in der Richtung des zweiten Endes erstreckende Vertiefung (12), die den Kanal für den Stab bildet, hat.

16. Aufnahmeteil von einem der Ansprüche 1 bis 15, wobei der Stabaufnahmeabschnitt (9) ein erstes Ende (9a) und ein zweites Ende (9b) hat, und ein Kopfaufnahmeabschnitt (16) auf der Seite des zweiten Endes (9b) angeordnet ist, und wobei der äußere Durchmesser des Kopfaufnahmeabschnitts (16) auf der Seite des zweiten Endes (9b) kleiner als der Durchmesser des Stabaufnahmeabschnitts (9) an dem zweiten Ende (9b) ist.

17. Knochenverankerungseinrichtung, die ein Aufnahmeteil gemäß einem der Ansprüche 1 bis 16 und ein Knochenverankerungselement (1) hat, das einen Schaft (2) zum Verankern in einem Knochen und einen Kopf (3), aufweist, und wobei der Kopf (3) vorzugsweise einen äußeren Außenflächenabschnitt hat, der kugelförmig ist.

18. Knochenverankerungsvorrichtung gemäß Anspruch 17, wobei ein Verschlusselement (7), vorzugsweise eine innere Schraube, zum Sichern des Stabs (6) in einer Vertiefung (12) vorgesehen ist.

## Revendications

1. Partie de réception destinée à recevoir une tige pour le couplage de la tige à un élément d'ancrage osseux, la partie de réception comportant
un corps de partie de réception (5) avec
une partie de réception de tige (9) avec un canal (12) pour recevoir la tige, et
une partie de réception de tête (16) avec une surface externe qui est globalement cylindrique et a un diamètre externe (d₁) pour loger une tête (3) de l'élément d'ancrage osseux, la partie de réception tête ayant une extrémité ouverte (17) et étant flexible, de manière à permettre l'introduction et le serrage de la tête, la partie de réception de tête (16) ayant une première partie de surface extérieure (16a, 16a', 16a") avec un plus grand diamètre externe (d₂) supérieur au diamètre externe (d₁) de la partie de réception de tête (16), une partie cylindrique (16b) adjacente à l'extrémité ouverte (17) et ayant un diamètre externe (d₃) supérieur au diamètre externe (d₁) de la partie de réception de tête (16) et inférieur au diamètre externe (d₂) de la première partie de surface extérieure (16a, 16a', 16a''), et un axe central (C) s'étendant à travers l'extrémité ouverte (17) ; et
un anneau de verrouillage (8) entourant la partie de réception de tête (16), l'anneau de verrouillage ayant une première partie de surface intérieure (83, 83', 83") qui s'engage avec la première partie de surface extérieure (16a, 16a', 16a'') dans une première région de contact pour serrer la tête (3) et une première partie cylindrique creuse (81), adjacente à une extrémité inférieure (8b) de l'anneau de verrouillage (8), avec un diamètre interne qui est légèrement inférieur au diamètre externe (d₃) de la partie cylindrique (16b) pour s'engager dans un raccordement à ajustement serré ;
dans laquelle au moins l'une des parties de surface extérieure et intérieure est une partie de surface cylindrique (83, 16a") et l'autre partie est soit une partie de surface cylindrique (83" , 16a ") ou une partie bombée de forme annulaire (16a, 83') et où l'anneau de verrouillage (8) est mobile vers l'extrémité ouverte (17) lors de l'exercice d'une pression sur celui-ci par l'intermédiaire de la tige (6) pour serrer la tête.

2. Partie de réception de la revendication 1, dans laquelle un diamètre interne de la première partie de surface intérieure (83, 83', 83") au niveau de la première région de contact est inférieur au diamètre externe de la première partie de surface extérieure (16a, 16a', 16a") au niveau de la première région de contact pour réaliser un raccordement à ajustement serré.

3. Partie de réception de la revendication 1 ou 2, dans laquelle la partie de réception de tête (16) a la partie cylindrique (16b) en tant que deuxième partie de surface extérieure qui est séparée de la première partie de surface extérieure (16a, 16a', 16a") dans une direction axiale et qui s'engage avec la première partie cylindrique creuse (81) en tant que deuxième partie de surface intérieure de l'anneau de verrouillage qui est séparée de la première partie de surface intérieure (83, 83', 83") dans une direction axiale pour former une deuxième région de contact.

4. Partie de réception de la revendication 3, dans laquelle la deuxième partie de surface extérieure est une partie de surface cylindrique (16b).

5. Partie de réception de la revendication 3 ou 4, dans laquelle la deuxième partie de surface intérieure est une partie de surface cylindrique (81).

6. Partie de réception de l'une des revendications 3 à 5, dans laquelle la deuxième région de contact est plus proche de l'axe central (C) dans une direction radiale que la première région de contact.

7. Partie de réception de l'une des revendications 3 à 5, dans laquelle la deuxième région de contact a la même distance de l'axe central (C) que la première région de contact.

8. Partie de réception de l'une des revendications 3 à 7, dans laquelle la deuxième région de contact est adjacente à l'extrémité ouverte (17).

9. Partie de réception de l'une des revendications 1 à 8, dans laquelle la première partie de surface extérieure (16a") et la deuxième partie de surface extérieure (83") sont toutes les deux des parties de surface cylindrique.

10. Partie de réception de l'une des revendications 1 à 8, dans laquelle la première partie de surface extérieure (16a) est une partie bombée de forme annulaire et la première partie de surface intérieure (83) est une partie de surface cylindrique.

11. Partie de réception de l'une des revendications 1 à 8, dans laquelle la première partie de surface extérieure (16a') est une partie de surface cylindrique et la première partie de surface intérieure (83') est une partie bombée de forme annulaire.

12. Partie de réception de l'une des revendications 3 à 11, dans laquelle une rainure (16c) est prévue entre la première partie de surface extérieure (16a, 16a', 16a") et une deuxième partie de surface extérieure (83, 83', 83") dans laquelle l'anneau de verrouillage peut s'engager lorsqu'il est dans une position d'insertion, dans laquelle la tête peut être insérée dans la partie de réception de tête (16) et retirée de celle-ci.

13. Partie de réception de l'une des revendications 1 à 12, dans laquelle l'anneau de verrouillage (8) peut prendre une position de pré-verrouillage, dans laquelle la tête peut pivoter dans la partie de réception de tête mais ne peut pas être retirée de la partie de réception de tête.

14. Partie de réception de l'une des revendications 1 à 13, dans laquelle l'anneau de verrouillage (8) peut prendre une position de verrouillage, dans laquelle la première partie de surface extérieure (16a, 16a', 16a") entre en contact avec la première partie de surface intérieure (83, 83', 83").

15. Partie de réception de l'une des revendications 1 à 14, dans laquelle la partie de réception de tige (9) a une première extrémité (9a) et une deuxième extrémité (9b) et un évidement (12) s'étendant à partir de la première extrémité dans la direction de la deuxième extrémité, qui forme le canal pour la tige.

16. Partie de réception de l'une des revendications 1 à 15, dans laquelle la partie de réception de tige (9) a une première extrémité (9a) et une deuxième extrémité (9b) et une partie de réception de tête (16) est agencée au niveau du côté de la deuxième extrémité (9b) et dans laquelle le diamètre externe de la partie de réception de tête (16) au niveau du côté de la deuxième extrémité (9b) est inférieur au diamètre de la partie de réception de tige (9) au niveau de la deuxième extrémité (9b).

17. Dispositif d'ancrage osseux comprenant une partie de réception selon l'une des revendications 1 à 16 et un élément d'ancrage osseux (1) ayant un arbre (2) pour l'ancrage dans un os et une tête (3) et où la tête (3) a de préférence une partie de surface externe extérieure, qui est de forme sphérique.

18. Dispositif d'ancrage osseux selon la revendication 17, dans lequel un élément de fermeture (7), de préférence une vis interne, est prévu pour fixer la tige (6) dans un évidement (12).
